# EUROPEAN PATENT APPLICATION

(11) **EP 3 799 109 A1**
(43) Date of publication of application: **31.03.2021**
(21) Application number: 19806453.7
(22) Date of filing: 07.05.2019
(51) Int. Cl.: H01J 61/35, B82Y 20/00, H01J 65/00

(54) **LIGHT TRANSMISSIVE MATERIAL AND LAMP, AND GAS TREATING DEVICE AND GAS TREATING METHOD**

(30) Priority: 22.05.2018 JP 2018097745; 04.06.2018 JP 2018106701
(71) Applicant: Ushio Denki Kabushiki Kaisha, Tokyo 100-8150 (JP)
(72) Inventor: NAITO Keisuke, Tokyo 100-8150 (JP)
(74) Representative: Tomerius, Isabel
(86) International application number: PCT/JP2019/018223
(87) International publication number: WO 2019/225303

(57) **Abstract**

There is provided a light transmissive material capable of reducing the attachment of contaminants to its front surface, and easily removing contaminants even when the contaminants have been attached thereto. There are also provided a lamp including a light emitting tube formed of the light transmissive material as well as a gas treatment device and a gas treatment method utilizing the light transmissive material.

The light transmissive material according to the present invention includes a glass substrate having a front surface on which a surface layer formed of nano-silica particles is provided. The lamp according to the present invention includes a light emitting tube formed of the above-described light transmissive material, in which the surface layer is provided on an outer surface of the light emitting tube. The gas treatment device according to the present invention includes: a chamber having a treatment space through which a gas to be treated flows; and an ultraviolet lamp disposed so that at least a part of a light emitting tube is exposed to the treatment space. The light emitting tube in the ultraviolet lamp has an outer surface on which a surface layer formed of nano-silica particles is provided.

## Description

### TECHNICAL FIELD

The present invention relates to a light transmissive material and a lamp including a light emitting tube formed of the light transmissive material, and a gas treatment device and a gas treatment method for treating a gas containing a harmful substance by utilizing ultraviolet rays.

### BACKGROUND ART

Methods utilizing ultraviolet rays have been known in the conventional techniques as methods for treating gases containing harmful substances.

Patent Literature 1, for example, discloses a gas treatment device in which an ultraviolet lamp that radiates vacuum ultraviolet rays having a wavelength of not longer than 200 nm is disposed in a housing through which a gas to be treated flows. In such a gas treatment device, active species such as ozone or oxygen radicals are generated by irradiating oxygen, contained in the gas to be treated, with vacuum ultraviolet rays, and the resulting active species decompose harmful substances contained in the gas to be treated.

Patent Literature 2 discloses a gas treatment device in which a photocatalyst part and an ultraviolet lamp are disposed in a container through which a gas to be treated flows. In such a gas treatment device, a photocatalyst is activated by irradiating the photocatalyst part with ultraviolet rays, and harmful substances contained in the gas to be treated are decomposed by the activated photocatalyst.

In such a gas treatment device, however, since an outer surface of a light emitting tube in the ultraviolet lamp is brought into contact with the gas to be treated, contaminants such as dust, a water-soluble or oil-soluble organic substance contained in the gas to be treated, and decomposition products produced by ultraviolet rays or the photocatalyst are attached to the outer surface of the light emitting tube. When the ultraviolet lamp is configured to radiate vacuum ultraviolet rays having a wavelength of not longer than 200 nm, in particular, a degree of the hydrophilicity of the outer surface of the light emitting tube has been increased by the irradiation of vacuum ultraviolet rays. Thus, such tendency becomes prominent. As a result, ultraviolet rays radiated from the ultraviolet lamp are blocked by the outer surface of the light emitting tube, thereby raising a problem that the intensity of illumination of ultraviolet rays for the gas to be treated or photocatalyst to be irradiated with is lowered. Moreover, when such contaminants have been attached to the outer surface of the light emitting tube, it is difficult to remove the contaminants easily, thus significantly complicating a maintenance operation of the ultraviolet lamp.

Patent Literature 3 discloses a gas treatment device including: a casing through which a gas to be treated flows; a cylindrical ultraviolet transmissive window member provided in the casing; and an ultraviolet lamp housed in the ultraviolet transmissive window member. In such a gas treatment device, since the ultraviolet lamp is isolated from a passage of the gas to be treated by the ultraviolet transmissive window member, an outer surface of a light emitting tube in the ultraviolet lamp is prevented from being in contact with the gas to be treated. Thus, contaminants can be prevented from being attached to the outer surface of the light emitting tube in the ultraviolet lamp.

An outer surface of the ultraviolet transmissive window member, however, is brought into contact with the gas to be treated. Thus, contaminants or the like are attached to the said outer surface, thereby raising a problem that the intensity of illumination of ultraviolet rays for the gas to be treated or photocatalyst to be irradiated with is lowered.

Furthermore, without being limited to ultraviolet lamps used in gas treatment devices, any lamp used in an environment in which dust or contaminants are more likely to be attached thereto has a problem of reduction in the intensity of illumination of its light due to the attachment of the contaminants to an outer surface of a light emitting tube.

### CITATION LIST

### PATENT LITERATURE

Patent Literature 1: Japanese Patent Application Laid-Open No. 2012-207536
Patent Literature 2: Japanese Patent Application Laid-Open No. 2011-147874
Patent Literature 3: Japanese Patent Application Laid-Open No. 2001-170453

### SUMMARY OF INVENTION

### Technical Problem

The present invention has as its first object the provision of a light transmissive material capable of reducing the attachment of contaminants to its front surface, and easily removing contaminants even when the contaminants have been attached thereto.

The present invention has as its second object the provision of a lamp capable of reducing the attachment of contaminants to an outer surface of a light emitting tube, and easily removing contaminants even when the contaminants have been attached to the outer surface of the light emitting tube.

The present invention has as its third object the provision of a gas treatment device and a gas treatment method capable of reducing reduction in the intensity of illumination of ultraviolet rays due to the attachment of contaminants to an outer surface of a light emitting tube in an ultraviolet lamp or a front surface of an ultraviolet transmissive window member, and capable of easily removing contaminants even when the contaminants have been attached thereto.

### Solution to Problem

There is provided a light transmissive material according to the present invention including a glass substrate having a front surface on which a surface layer formed of nano-silica particles is provided.

In the light transmissive material of the present invention, the nano-silica particles may preferably have an average particle size of smaller than 100 nm.

The glass substrate may preferably be transmissive to an ultraviolet ray.

The glass substrate may preferably be formed of silica glass.

In such a light transmissive material, the nano-silica particles may preferably have an average particle size of not larger than 50 nm.

There is provided a lamp according to the present invention that includes a light emitting tube formed of the above-described light transmissive material, in which the surface layer is provided on an outer surface of the light emitting tube.

The lamp of the present invention may preferably be configured to radiate an ultraviolet ray.

The lamp may preferably be configured as an excimer discharge lamp that radiates a vacuum ultraviolet ray having a wavelength not longer than 200 nm.

There is provided a gas treatment device according to the present invention including: a chamber having a treatment space through which a gas to be treated flows; and an ultraviolet lamp disposed so that at least a part of a light emitting tube is exposed to the treatment space. The light emitting tube in the ultraviolet lamp has an outer surface on which a surface layer formed of nano-silica particles is provided.

In such a gas treatment device, the light emitting tube may preferably be formed of silica glass.

There is also provided a gas treatment device according to the present invention including: a chamber having a treatment space through which a gas to be treated flows; an ultraviolet transmissive window member disposed so that at least a part of a front surface of the ultraviolet transmissive window member is exposed to the treatment space; and an ultraviolet lamp that irradiates the treatment space with an ultraviolet ray via the ultraviolet transmissive window member. A surface layer formed of nano-silica particles is provided on the front surface of the ultraviolet transmissive window member exposed to the treatment space.

In such a gas treatment device, the ultraviolet transmissive window member may preferably be formed of silica glass.

In the gas treatment device of the present invention, the nano-silica particles may preferably have an average particle size of smaller than 100 nm.

The ultraviolet lamp may preferably be an excimer lamp.

The gas treatment device of the present invention may be configured to treat a gas to be treated containing a volatile organic compound.

There is provided a gas treatment method according to the present invention including irradiating a gas to be treated flowing through a treatment space with an ultraviolet ray using an ultraviolet lamp disposed so that at least a part of a light emitting tube is exposed to the treatment space. The light emitting tube in the ultraviolet lamp has an outer surface on which a surface layer formed of nano-silica particles is provided.

There is also provided a gas treatment method according to the present invention including irradiating a gas to be treated flowing through a treatment space with an ultraviolet ray via an ultraviolet transmissive window member disposed so that at least a part of a front surface of the ultraviolet transmissive window member is exposed to the treatment space. A surface layer formed of nano-silica particles is provided on the front surface of the ultraviolet transmissive window member exposed to the treatment space.

There is further provided a gas treatment method according to the present invention including irradiating a photocatalyst disposed in a treatment space through which a gas to be treated flows with an ultraviolet ray from an ultraviolet lamp disposed so that at least a part of a light emitting tube is exposed to the treatment space. The light emitting tube in the ultraviolet lamp has an outer surface on which a surface layer formed of nano-silica particles is provided.

There is further provided a gas treatment method according to the present invention including irradiating a photocatalyst disposed in a treatment space through which a gas to be treated flows with an ultraviolet ray via an ultraviolet transmissive window member disposed so that at least a part of a front surface of the ultraviolet transmissive window member is exposed to the treatment space. A surface layer formed of nano-silica particles is provided on the front surface of the ultraviolet transmissive window member exposed to the treatment space.

In the gas treatment method of the present invention, the gas to be treated may be a gas containing a volatile organic compound.

### ADVANTAGEOUS EFFECTS OF INVENTION

According to the light transmissive material of the present invention, since the surface layer formed of the nano-silica particles is provided on the front surface, the attachment of contaminants to the front surface can be reduced, and contaminants can be easily removed even when the contaminants have been attached thereto.

According to the lamp of the present invention, since the surface layer formed of the nano-silica particles is provided on the outer surface of the light emitting tube, the attachment of contaminants to the outer surface of the light emitting tube can be reduced, and contaminants can be easily removed even when the contaminants have been attached to the outer surface of the light emitting tube.

According to the gas treatment devices and the gas treatment methods of the present invention, since the surface layer formed of the nano-silica particles is provided on the outer surface of the light emitting tube in the ultraviolet lamp or the front surface of the ultraviolet transmissive window member exposed to the treatment space through which the gas to be treated flows, the attachment of contaminants to the outer surface of the light emitting tube in the ultraviolet lamp or the front surface of the ultraviolet transmissive window member can be reduced. Accordingly, reduction in the intensity of illumination of the ultraviolet ray due to the attachment of contaminants to the outer surface of the light emitting tube or the front surface of the ultraviolet transmissive window member can be reduced. Furthermore, even when contaminants have been attached to the outer surface of the light emitting tube or the front surface of the ultraviolet transmissive window member, such contaminants can be easily removed.

### BRIEF DESCRIPTION OF DRAWINGS

[FIG. 1] is an explanatory view illustrating the configuration of an example of a lamp according to the present invention.
[FIG. 2] is an explanatory sectional view illustrating the configuration of an example of a gas treatment device according to the present invention.
[FIG. 3] is an explanatory sectional view illustrating the configuration of another example of the gas treatment device according to the present invention.

### DESCRIPTION OF EMBODIMENTS

An embodiment of the present invention will be described below in detail. Light Transmissive Material:
A light transmissive material of the present invention includes a glass substrate having a front surface on which a surface layer formed of nano-silica particles is provided.

In the light transmissive material of the present invention, the surface layer formed of the nano-silica particles is not required to be formed over the entire front surface of the glass substrate. The surface layer may be formed only on a part of the front surface of the glass substrate that requires an antifouling property.

In the light transmissive material of the present invention, the glass substrate is not limited to any particular form. Various forms, such as a plate shape, a tube shape, a double-tube shape and a hollow box shape, may be adopted depending on the uses of the light transmissive material.

Glass that constitutes the glass substrate is not limited to any particular glass as long as the glass is transmissive to light having a target wavelength. If a light transmissive material transmissive to ultraviolet rays is produced, however, silica glass (fused silica glass or synthetic silica glass), sapphire glass, magnesium fluoride glass or the like may preferably be used. If a light transmissive material transmissive to vacuum ultraviolet rays having a wavelength of not longer than 200 nm is produced, synthetic silica glass, sapphire glass, magnesium fluoride glass or the like may preferably be used.

The glass substrate is not limited to any particular thickness as long as the thickness of the glass substrate can transmit light having a target wavelength. An example of the thickness of the glass substrate is 0.5 to 2 mm.

The nano-silica particles that constitute the surface layer preferably may have an average particle size of smaller than 100 nm. Although the lower limit of the average particle size of the nano-silica particles is not limited to any particular value, nano-silica particles having an average particle size of not smaller than 5 nm may preferably be used from the viewpoint of their production.

If a light transmissive material transmissive to vacuum ultraviolet rays having a wavelength of not longer than 200 nm is produced, nano-silica particles may preferably have an average particle size of not larger than 50 nm, more preferably not larger than 30 nm, and yet more preferably not larger than 20 nm.

An excessively large average particle size of nano-silica particles may possibly deteriorate the transmissive property to light having a target wavelength.

As examples of a method of forming the surface layer on the front surface of the glass substrate, may be used a dry method according to which nano-silica particles are pressed against the front surface of the glass substrate, and a wet method according to which a dispersion liquid including dispersed nano-silica particles is applied to the front surface of the glass substrate and then dried.

As specific examples of the dry method, may be mentioned a method according to which nano-silica particles are pressed against the front surface of the glass substrate by placing the glass substrate and the nano-silica particles in a cylindrical container, disposing the container with a cylindrical axis thereof aligning with the horizontal direction and then rotating the container with the cylindrical axis being used as a central axis as in a ball mill, for example, and a method according to which nano-silica particles are pressed against the front surface of the glass substrate by a roller.

As examples of a liquid dispersion medium for preparing a dispersion liquid in the wet method, may be used hydrophilic organic solvents such as isopropyl alcohol, methyl alcohol, ethylene glycol, ethylene glycol mono-n-propyl ether, propylene glycol monomethyl ether and dimethylacetamide, and hydrophobic organic solvents such as methyl ethyl ketone, methyl isobutyl ketone, cyclohexane, ethyl acetate, propylene glycol monomethyl ether acetate and toluene.

The percentage of the nano-silica particles in the dispersion liquid is 15 to 50% by mass, for example.

As examples of a method of applying the dispersion liquid to the front surface of the glass substrate, may be used a dip method, a spray coating method, an application method by an ink-jet printer and a screen printing method.

According to such a light transmissive material, the attachment of contaminants to the front surface can be reduced since the surface layer formed of the nano-silica particles is provided on the front surface. Even when contaminants have been attached to the front surface, such contaminants can be easily removed.

The light transmissive material of the present invention can be employed for a variety of uses requiring an antifouling property. Such a light transmissive material can be used as a light emitting tube of a lamp, an outer tube for protecting a lamp or a light transmissive window member that transmits light from a lamp, for example.

### Lamp:

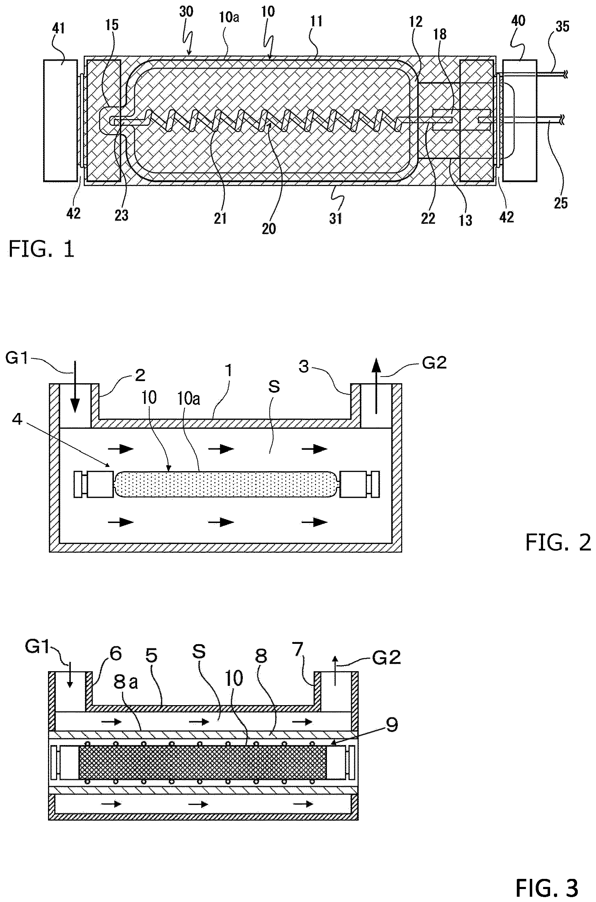

FIG. 1 is an explanatory view illustrating the configuration of an example of a lamp according to the present invention.

This lamp is an excimer discharge lamp that radiates vacuum ultraviolet rays having a wavelength of not longer than 200 nm, and has a light emitting tube 10 formed of the above-described light transmissive material. Specifically, the light emitting tube 10 is configured to include a tubular glass substrate, which constitutes the light emitting tube 10, having an outer surface 10a on which a surface layer formed of nano-silica particles is provided.

The surface layer formed of the nano-silica particles is not required to be formed over the entire outer surface of the light emitting tube 10. The surface layer may be formed only in a part of the outer surface 10a of the light emitting tube 10 that requires an antifouling property.

The light emitting tube 10 in the illustrated example includes: a light emitting part 11 having a circular tube shape; and a sealing part 13 continuous with one end of the light emitting part 11 via a reduced diameter part 12. A tip part 15, which is a remaining part of an exhaust path for exhausting air in the light emitting tube 10 in a process of manufacturing the lamp, is formed at the other end of the light emitting tube 10.

The sealing part 13 has what is called a foil sealing structure in which a metal foil 18 is buried and hermetically sealed. For example, the sealing part 13 is formed by heating an end of a light emitting tube forming material that constitutes the light emitting tube 10 and then collapsing the end with a pincher (pinch-sealed structure) .

Cylindrical base members 40 and 41 formed of, for example, inorganic insulating ceramic (for example, alumina) are provided at both ends of the light emitting tube 10. The base members 40 and 41 are fixed to the light emitting tube 10 via an inorganic adhesive, for example. Outer circumferential surfaces of the base members 40 and 41 are each provided with a groove part 42 extending along its whole circumference in a circumferential direction thereof.

Inside the light emitting tube 10, a light emitting gas for generating excimer is encapsulated, and an inner electrode 20 is disposed so as to extend along a tube axis of the light-emitting tube 10.

As examples of the light emitting gas, may be used noble gases, such as a xenon gas (Xe), an argon gas (Ar) and a krypton gas (Kr), each having a function as a discharge medium for generating excimer molecules by excimer discharge. Together with such a noble gas, a halogen gas such as a fluorine gas (F), a chlorine gas (Cl), an iodine gas (I) or a bromine gas (Br) may be encapsulated as a discharge medium as needed.

The inner electrode 20 is formed of a metal having heat resistance, such as tungsten. The inner electrode 20 includes: a coil part 21 formed by winding a metal element wire in a coil shape; and lead parts 22 and 23 provided at both ends of the coil part 21 so as to extend in a generally straight manner. The inner electrode 20 is disposed so that a central axis of the coil part 21 coincides with the tube axis of the light emitting tube 10. The other end of the other lead part 23 is located in the tip part 15, and one end of the one lead part 22 is electrically connected to the other end of the metal foil 18 buried in the sealing part 13.

The other end of an external lead 25, which extends so as to protrude outwardly in the tube axis direction from an outer end of the sealing part 13, is electrically connected to one end of the metal foil 18. In this manner, the inner electrode 20 is electrically connected to the external lead 25 via the metal foil 18.

A netted outer electrode 30 is provided on the outer surface of the light emitting tube 10 so as to extend along the tube axis direction of the light emitting tube 10. The outer electrode 30 is connected to a power source (not shown) via a wire 35 extending so as to pass through the one base member 40. The outer electrode 30 serves as a ground electrode, for example.

The outer electrode 30 in this example is formed by, for example, a netted electrode forming member obtained by braiding a plurality of electrically-conductive metal element wires in a cylindrical shape (hereinafter, referred to also as a "netted electrode forming member"). A diameter of the metal element wire that constitutes the netted electrode forming member 31 is φ0.01 to φ1.0 mm, for example.

In such a lamp, a high-frequency high voltage is applied between the inner electrode 20 and the outer electrode 30 by the power source not shown, thereby generating excimer discharge in an internal space of the light emitting tube 10. The excimer discharge generates excimer molecules, and vacuum ultraviolet light emitted from the excimer molecules is transmitted through the light emitting tube 10 and then radiated via mesh openings of the netted electrode forming member 31 that constitutes the outer electrode 30.

According to the above-described lamp, since the surface layer formed of the nano-silica particles is provided on the outer surface of the light emitting tube 10, the attachment of contaminants to the outer surface of the light emitting tube 10 can be reduced. Accordingly, reduction in the intensity of illumination due to the attachment of contaminants to the outer surface of the light emitting tube 10 can be reduced. Moreover, since contaminants are less likely to be attached to the outer surface of the light emitting tube 10, the frequency of washing the outer surface of the light emitting tube 10 decreases. Furthermore, even when contaminants have been attached to the outer surface of the light emitting tube 10, such contaminants can be easily removed.

The lamp of the present invention is not limited to the above-described embodiment, and various modifications can be made thereto.

For example, the lamp of the present invention is not limited to the excimer discharge lamp, but can be configured as any of discharge lamps such as low-pressure mercury lamps, halogen lamps and other various lamps.

### Gas Treatment Device and Gas Treatment Method:

FIG. 2 is an explanatory sectional view illustrating the configuration of an example of a gas treatment device according to the present invention.

According to the gas treatment device shown in FIG. 2, active species such as ozone or oxygen radicals are generated by irradiating oxygen in a gas to be treated with vacuum ultraviolet rays having a wavelength of not longer than 200 nm, and the resulting active species decompose harmful substances contained in the gas to be treated.

The gas treatment device has a cylindrical chamber 1 having a treatment space S through which a gas G1 to be treated flows. A gas introducing tube 2 for introducing the gas G1 to be treated into the treatment space S is provided on one end side of a peripheral wall of the chamber 1. A gas introducing tube 3 for discharging a treated gas G2 from the treatment space S is provided on the other end side of the peripheral wall of the chamber 1.

An excimer discharge lamp 4 having a light emitting tube 10 with a straight tube shape is disposed in the treatment space S of the chamber 1 so as to extend along a cylindrical axis of the chamber 1.

An excimer discharge lamp having the configuration shown in FIG. 1 can be used as the excimer discharge lamp 4. The surface layer formed of the nano-silica particles that is provided on the outer surface 10a of the light emitting tube 10 is not required to be formed over the entire outer surface of the light emitting tube 10. The surface layer may be formed only in a part of the outer surface 10a of the light emitting tube 10 that requires an antifouling property, i.e., a region exposed to the treatment space S.

In such a gas treatment device, the gas G1 to be treated is introduced into the treatment space S through the gas introducing tube 2 of the chamber 1. In the excimer discharge lamp 4, a high-frequency high voltage is applied between the inner electrode 20 and the outer electrode 30 of the excimer discharge lamp 4 by the power source not shown, thereby generating excimer discharge in the internal space of the light emitting tube 10. The excimer discharge generates excimer molecules, and vacuum ultraviolet light emitted from the excimer molecules is transmitted through the light emitting tube 10 and then radiated via the mesh openings of the netted electrode forming member 31 that constitutes the outer electrode 30. By irradiating oxygen in the gas G1 to be treated with such vacuum ultraviolet rays, active species such as ozone or oxygen radicals are generated. The resulting active species decompose harmful substances contained in the gas to be treated, for example, volatile organic compounds, thus completing the gas treatment. The treated gas G2 is then discharged from the treatment space S to the outside through the gas discharging tube 3.

In the foregoing configuration, a clearance between an inner surface of the chamber 1 and the outer surface 10a of the light emitting tube 10 in the excimer discharge lamp 4 is preferably 5 to 30 mm. If such a clearance is excessively small, the flow rate of the gas G1 to be treated on the front surface of the light emitting tube 10 becomes too large, thus possibly failing to provide sufficient energy to the gas G1 to be treated. If such a clearance is excessively large, on the other hand, ultraviolet rays from the excimer discharge lamp 4 are absorbed by the gas G1 to be treated, thus preventing a sufficient amount of ultraviolet rays from reaching a region of the treatment space S farthest from the front surface of the light emitting tube 10, for example, a region near the inner surface of the chamber 1. Consequently, there is a possibility that harmful substances in the gas G1 to be treated flowing through such a region are discharged from the treatment space S without being sufficiently decomposed.

The intensity of illumination of the vacuum ultraviolet rays from the excimer discharge lamp 4 to the gas to be treated in the treatment space S is 20 to 100 mW/cm², for example.

The flow rate of the gas to be treated flowing through the treatment space S is 1 to 1000 L/min, for example, although the flow rate is set in consideration of the residence time of the gas to be treated in the treatment space S.

According to the above-described gas treatment device, since the surface layer formed of the nano-silica particles is provided on the outer surface 10a of the light emitting tube 10 in the excimer discharge lamp 4, the attachment of contaminants to the outer surface 10a of the light emitting tube 10 can be reduced. Accordingly, reduction in the intensity of illumination of ultraviolet rays due to the attachment of contaminants to the outer surface 10a of the light emitting tube 10 can be reduced. Moreover, since contaminants are less likely to be attached to the outer surface 10a of the light emitting tube 10, the frequency of washing the outer surface 10a of the light emitting tube 10 decreases. Furthermore, even when contaminants have been attached to the outer surface 10a of the light emitting tube 10, such contaminants can be easily removed.

FIG. 3 is an explanatory sectional view illustrating the configuration of another example of a gas treatment device according to the present invention. According to the gas treatment device shown in FIG. 3, active species such as ozone or oxygen radicals are generated by irradiating oxygen in a gas to be treated with vacuum ultraviolet rays having a wavelength of not longer than 200 nm, and the resulting active species decompose harmful substances contained in the gas to be treated.

This gas treatment device has a cylindrical chamber 5 having a treatment space S through which a gas G1 to be treated flows. A gas introducing tube 6 for introducing the gas G1 to be treated into the treatment space S is provided on one end side of a peripheral wall of the chamber 5. A gas introducing tube 7 for discharging a treated gas G2 from the treatment space S is provided on the other end side of the peripheral wall of the chamber 5.

A tubular ultraviolet transmissive window member 8 is disposed in the treatment space S of the chamber 5 so as to pass through both end walls of the chamber 5 and extend along a cylindrical axis of the chamber 5.

An excimer discharge lamp 9 including a light emitting tube 10 having a straight tube shape is disposed inside the ultraviolet transmissive window member 8 so as to extend along a tube axis of the ultraviolet transmissive window member 8. That is, the excimer discharge lamp 9 is disposed in a space isolated from the treatment space S through which the gas G1 to be treated flows by the ultraviolet transmissive window member 8. The excimer discharge lamp 9 has the same configuration as that of the excimer discharge lamp 4 shown in FIG. 1 except that no surface layer formed of nano-silica particles is provided on an outer surface 10a of the light emitting tube 10.

The ultraviolet transmissive window member 8 is formed of a glass material transmissive to ultraviolet rays from the excimer discharge lamp 9. As preferable examples of such a glass material, may be used silica glass (fused silica glass or synthetic silica glass), sapphire glass and magnesium fluoride glass.

On an outer surface 8a of the ultraviolet transmissive window member 8 exposed to the treatment space S, provided is a surface layer formed of nano-silica particles. The surface layer provided on the ultraviolet transmissive window member 8 has the same configuration as that of the surface layer provided on the outer surface 10a of the light emitting tube 10 in the excimer discharge lamp 4 shown in FIG. 2.

The inside of the ultraviolet transmissive window member 8 is purged with an inert gas such as a nitrogen gas.

In such a gas treatment device, the gas G1 to be treated is introduced into the treatment space S through the gas introducing tube 6 of the chamber 5. By irradiating oxygen in the gas G1 to be treated with vacuum ultraviolet rays from the excimer discharge lamp 9 via the ultraviolet transmissive window member 8, active species such as ozone or oxygen radicals are generated. The resulting active species decompose harmful substances contained in the gas to be treated, for example, volatile organic compounds, thus completing the gas treatment. The treated gas G2 is then discharged from the treatment space S to the outside through the gas discharging tube 3.

In the foregoing configuration, a clearance between an inner surface of the chamber 5 and the outer surface 8a of the ultraviolet transmissive window member 8 may preferably be 5 to 30 mm.

The intensity of illumination of the vacuum ultraviolet rays from the excimer discharge lamp 9 to the gas to be treated in the treatment space S and the flow rate of the gas to be treated flowing through the treatment space S have the same conditions as those in the gas treatment device shown in FIG. 1.

According to the above-described gas treatment device, since the surface layer formed of the nano-silica particles is provided on the outer surface 8a of the ultraviolet transmissive window member 8 exposed to the treatment space S, the attachment of contaminants to the outer surface 8a of the ultraviolet transmissive window member 8 can be reduced. Accordingly, reduction in the intensity of illumination of ultraviolet rays due to the attachment of contaminants to the outer surface 8a of the ultraviolet transmissive window member 8 can be reduced. Moreover, since contaminants are less likely to be attached to the outer surface 8a of the ultraviolet transmissive window member 8, the frequency of washing the outer surface 8a of the ultraviolet transmissive window member 8 decreases. Furthermore, even when contaminants have been attached to the outer surface 8a of the ultraviolet transmissive window member 8, such contaminants can be easily removed.

The gas treatment device and the gas treatment method of the present invention are not limited to the above-described embodiments, and various modifications can be made thereto.
(1) Instead of the excimer discharge lamp, a low-pressure mercury lamp may be used as an ultraviolet lamp.
(2) According to the above-described embodiments, harmful substances contained in a gas to be treated are decomposed by active species, such as ozone or oxygen radicals, generated by irradiating oxygen contained in the gas to be treated with vacuum ultraviolet rays. Harmful substances contained in a gas to be treated, however, may be decomposed by a photocatalyst activated by the irradiation of ultraviolet rays. In such an embodiment, a photocatalyst including titanium dioxide, for example, may be disposed at an appropriate place in a treatment space, and a lamp that radiates ultraviolet rays capable of activating the photocatalyst may be used as an ultraviolet lamp. As examples of a means that disposes the photocatalyst in the treatment space, may be mentioned a means that carries the photocatalyst on an inner wall surface of a chamber and a means that disposes a carrier with the photocatalyst being carried thereby in the treatment space.
(3) The gas to be treated is not limited to a gas containing a volatile organic compound. The gas to be treated may be any gas containing a harmful substance capable of being decomposed by active species such as ozone or oxygen radicals, or an activated photocatalyst.
(4) In the gas treatment device shown in FIG. 2, the ultraviolet lamp (excimer discharge lamp) is disposed so that the entire front surface of the light emitting part in the light emitting tube is exposed to the treatment space. The ultraviolet lamp, however, may be disposed so that only a part of the front surface of the light emitting part is exposed to the treatment space.

### Examples:

### Example 1:

An excimer discharge lamp having the following specification was produced in accordance with the configuration shown in FIG. 1.
Light emitting tube (10):
   Material of glass substrate = silica glass
   Material of surface layer = nano-silica particles having average particle size of 10 nm
   Outer diameter of light emitting part (11) = 16 mm
   Inner diameter of light emitting part (11) = 14 mm
   Length of light emitting part (11) = 130 mm
Inner electrode (20):
   Material = tungsten
   Diameter of element wire = 0.3 mm
Outer electrode (30):
   Material = stainless steel (SUS304)
   Diameter of element wire = 0.2 mm
Encapsulated gas:
   Type of gas: xenon gas
   Charged pressure: 20 kPa
   Rated voltage: 4 kV

In the foregoing configuration, the surface layer of the light emitting tube was formed as follows.

The nano-silica particles were pressed against a front surface of the glass substrate by placing the glass substrate that constitutes the light emitting tube and 1 g of the nano-silica particles having an average particle size of 10 nm in a cylindrical container having an inner diameter of 90 mm and a length of 150 mm, disposing the container with a cylindrical axis thereof aligning with the horizontal direction and then rotating the container at 200 rpm for 10 minutes with the cylindrical axis being used as a central axis. In this manner, the surface layer was provided on an outer surface of the glass substrate.

A surface analysis was made on the obtained outer surface of the light emitting tube using an atomic force microscope (manufactured by KEYENCE CORPORATION, product number: VN-8010), and its surface roughness Ra was measured. The result was 5 nm.

### Example 2:

An excimer discharge lamp having the same specification as that in Example 1 was produced except that nano-silica particles having an average particle size of 50 nm were used.

The surface roughness Ra of an outer surface of a light emitting tube was measured in the same manner as in Example 1, and the result was 12.1 nm.

### Example 3:

An excimer discharge lamp having the same specification as that in Example 1 was produced except that a surface layer of a light emitting tube was formed as follows.

A dispersion liquid obtained by dispersing nano-silica particles having an average particle size of 10 nm at 30% by mass was applied to an outer surface of a glass substrate, which constituted the light emitting tube, using the dip method, and left for 72 hours to be dried. In this manner, the surface layer was provided on the outer surface of the glass substrate.

### Comparative Example 1:

An excimer discharge lamp having the same specification as Example 1 was produced except that no surface layer formed of nano-silica particles was provided.

The surface roughness Ra of an outer surface of a light emitting tube was measured in the same manner as in Example 1, and the result was 1.7 nm.

### Test 1:

The intensity of illumination of ultraviolet rays radiated from each of the excimer discharge lamps according to Example 1, Example 2 and Comparative Example 1 was measured. Taking the intensity of illumination of ultraviolet rays from the excimer discharge lamp of Comparative Example 1 as 100, a relative value was obtained about the intensity of illumination of ultraviolet rays from each of the excimer discharge lamps according to Example 1 and Example 2. The relative values of the excimer discharge lamps according to Example 1 and Example 2 were 99.8 and 49.5, respectively.

### Test 2:

A powder contaminant (JIS test power class 11 was used), a water-soluble contaminant (JIS L 1919, Table 3) and an oil-soluble contaminant (JIS L 1919, Table 4) were attached to each of the excimer discharge lamps according to Example 1, Example 2 and Comparative Example 1. Using a method of attaching these contaminants in conformity with JIS L 1919, the contaminants were attached, and then exposed to running water for one minute. Evaluations were made on the antifouling property and washability of each of the light emitting tubes against these contaminants.

The results are shown in the following Table 1.

| | POWDER CONTAMINANT | | WATER - SOLUBLE CONTAMINANT | | OIL-SOLUBLE CONTAMINANT | |
|---|---|---|---|---|---|---|
| | ANTIFOULING PROPERTY | WASHABILITY | ANTIFOULING PROPERTY | WASHABILITY | ANTIFOULING PROPERTY | WASHABILITY |
| EXAMPLE 1 | Good | Good | Good | Good | Good | Good |
| EXAMPLE 2 | Good | Good | Good | Good | Good | Good |
| COMPARATIVE EXAMPLE 1 | Fair | Poor | Fair | Fair | Fair | Poor |

### Test 3:

Two petri dishes (having a diameter of about 37 mm) filled with toluene and the excimer discharge lamps according to Example 1, Example 3 and Comparative Example 1 were placed in a desiccator with 100L capacity. The toluene concentration in the 100-L desiccator was about 40 ppm. Thereafter, the excimer discharge lamps according to Example 1, Example 3 and Comparative Example 1 were lighted. After 24 hours and after 48 hours since the start of such lighting, the intensity of illumination of ultraviolet rays from each of the excimer discharge lamps according to Example 1, Example 3 and Comparative Example 1 was measured, and retention rates with respect to the initial intensity of illumination before the test were obtained.

The results are shown in the following Table 2.

**[Table 2]**

| | AFTER 24 HOURS | AFTER 48 HOURS |
|---|---|---|
| EXAMPLE 1 | 95% | 90% |
| EXAMPLE 3 | 91% | 88% |
| COMPARATIVE EXAMPLE 1 | 74% | 74% |

As is apparent from the results of Table 1, it was confirmed that the excimer discharge lamps according to the examples of the present invention could reduce the attachment of the contaminants to the outer surfaces of the light emitting tubes and contaminants could be easily removed even when such contaminants had been attached to the outer surfaces of the light emitting tubes.

The light emitting tube of the excimer discharge lamp according to Comparative Example 1, in contrast, exhibited a low antifouling property and low washability to any of the powder contaminant, the water-soluble contaminant and the oil-soluble contaminant.

### REFERENCE SIGNS LIST

- 1: chamber
- 2: gas introducing tube
- 3: gas discharging tube
- 4: excimer discharge lamp
- 5: chamber
- 6: gas introducing tube
- 7: gas discharging tube
- 8: ultraviolet transmissive window member
- 8a: outer surface
- 9: excimer discharge lamp
- 10: light emitting tube
- 10a: outer surface
- 11: light emitting part
- 12: reduced diameter part
- 13: sealing part
- 15: tip part
- 18: metal foil
- 20: inner electrode
- 21: coil part
- 22: one lead part
- 23: the other lead part
- 25: external lead
- 30: outer electrode
- 31: netted electrode forming member
- 35: wire
- 40: one base member
- 41: the other base member
- 42: groove part
- G1: gas to be treated
- G2: treated gas
- S: treatment space

## Claims

1. A light transmissive material comprising a glass substrate having a front surface on which a surface layer formed of nano-silica particles is provided.

2. The light transmissive material according to claim 1, wherein the nano-silica particles have an average particle size of smaller than 100 nm.

3. The light transmissive material according to claim 1 or 2, wherein the glass substrate is transmissive to an ultraviolet ray.

4. The light transmissive material according to claim 3, wherein the glass substrate is formed of silica glass.

5. The light transmissive material according to claim 4, wherein the nano-silica particles have an average particle size of not larger than 50 nm.

6. A lamp comprising a light emitting tube formed of the light transmissive material according to any one of claims 1 to 5, wherein the surface layer is formed on an outer surface of the light emitting tube.

7. The lamp according to claim 6, configured to radiate an ultraviolet ray.

8. The lamp according to claim 7, configured as an excimer discharge lamp that radiates a vacuum ultraviolet ray having a wavelength not longer than 200 nm.

9. A gas treatment device comprising: a chamber having a treatment space through which a gas to be treated flows; and an ultraviolet lamp disposed so that at least a part of a light emitting tube is exposed to the treatment space, wherein
the light emitting tube in the ultraviolet lamp has an outer surface on which a surface layer formed of nano-silica particles is provided.

10. The gas treatment device according to claim 9, wherein the light emitting tube is formed of silica glass.

11. A gas treatment device comprising: a chamber having a treatment space through which a gas to be treated flows; an ultraviolet transmissive window member disposed so that at least a part of a front surface of the ultraviolet transmissive window member is exposed to the treatment space; and an ultraviolet lamp that irradiates the treatment space with an ultraviolet ray via the ultraviolet transmissive window member, wherein
a surface layer formed of nano-silica particles is provided on the front surface of the ultraviolet transmissive window member exposed to the treatment space.

12. The gas treatment device according to claim 11, wherein the ultraviolet transmissive window member is formed of silica glass.

13. The gas treatment device according to any one of claims 9 to 12, wherein the nano-silica particles have an average particle size of smaller than 100 nm.

14. The gas treatment device according to claim 10 or 12, wherein the ultraviolet lamp is an excimer lamp.

15. The gas treatment device according to any one of claims 9 to 14, configured to treat a gas to be treated containing a volatile organic compound.

16. A gas treatment method comprising: irradiating a gas to be treated flowing through a treatment space with an ultraviolet ray using an ultraviolet lamp disposed so that at least a part of a light emitting tube is exposed to the treatment space, wherein
the light emitting tube in the ultraviolet lamp has an outer surface on which a surface layer formed of nano-silica particles is provided.

17. A gas treatment method comprising: irradiating a gas to be treated flowing through a treatment space with an ultraviolet ray via an ultraviolet transmissive window member disposed so that at least a part of a front surface of the ultraviolet transmissive window member is exposed to the treatment space, wherein
a surface layer formed of nano-silica particles is provided on the front surface of the ultraviolet transmissive window member exposed to the treatment space.

18. A gas treatment method comprising: irradiating a photocatalyst disposed in a treatment space through which a gas to be treated flows with an ultraviolet ray from an ultraviolet lamp disposed so that at least a part of a light emitting tube is exposed to the treatment space, wherein
the light emitting tube in the ultraviolet lamp has an outer surface on which a surface layer formed of nano-silica particles is provided.

19. A gas treatment method comprising: irradiating a photocatalyst disposed in a treatment space through which a gas to be treated flows with an ultraviolet ray via an ultraviolet transmissive window member disposed so that at least a part of a front surface of the ultraviolet transmissive window member is exposed to the treatment space, wherein
a surface layer formed of nano-silica particles is provided on the front surface of the ultraviolet transmissive window member exposed to the treatment space.

20. The gas treatment method according to any one of claims 16 to 19, wherein the gas to be treated is a gas containing a volatile organic compound.
